# EUROPEAN PATENT APPLICATION

(11) **EP 1 541 085 A1**
(43) Date of publication of application: **15.06.2005**
(21) Application number: 03748536.4
(22) Date of filing: 18.09.2003
(51) Int. Cl.: A61B 5/11, A47K 13/30, A61G 12/00

(54) **PHYSICAL MOVEMENT EVALUATION DEVICE AND PHYSICAL MOVEMENT EVALUATION SYSTEM**

(30) Priority: 19.09.2002 JP 2002273679
(71) Applicant: MATSUSHITA ELECTRIC INDUSTRIAL CO., LTD., Kadoma-shi, Osaka 571-8501 (JP)
(72) Inventor: TANAKA, Shinji, Ibaraki-shi, Osaka 567-0042 (JP); INOUE, Shigeyuki, Kyotanabe-shi, Kyoto 610-0357 (JP); YAMAMOTO, Hiroshi, Shijonawate-shi, Osaka 575-0013 (JP)
(74) Representative: Grünecker, Kinkeldey, Stockmair & Schwanhäusser Anwaltssozietät
(86) International application number: PCT/JP2003/011864
(87) International publication number: WO 2004/026138

(57) **Abstract**

A body motion evaluation apparatus 11 of the present invention is an apparatus capable of being attached to a human body, comprising: acceleration detecting means 12 of detecting information about an acceleration produced with a motion of a human body; calculating means 13 of performing predetermined calculation based on the detected information about the acceleration; calculation controlling means 14 of setting a predetermined calculation time period in the calculation; and signal receiving means 17 of receiving from outside a first behavior detection signal including information about detection of a start or end of a first predetermined behavior of the human body, wherein the calculation controlling means 14 determines a start point of the calculation based on the first behavior detection signal received by the signal receiving means 17, and sets as the calculation time period a time period from the start point until predetermined time elapses.

## Description

### Technical Field

The present invention relates to a body motion evaluation apparatus and a body motion evaluation system capable of being used in rehabilitation or the like.

### Background Art

There exist a large number of persons who have undergone operations for osteopathy and the like, and need rehabilitation, and it is said that the speed of recovery from such a disease depends on self-active training by a patient.

Furthermore, in recent years, for example, it has been increasingly required that the health of aged persons living alone be capable of being checked by doctors or relatives in remote areas with the advent of an aging society . Furthermore, aside from this, it has been increasingly required that management of the physical condition of an aged inpatient in hospital be conducted more minutely.

Furthermore, even for normal healthy people, the number of anemic persons has increased with a change in eating habits, and dizziness or giddiness on standing up may cause a violent fall, resulting in a dangerous situation.

Furthermore, a side from the cause described above, causes of equilibration disorders such as dizziness may include ear diseases such as vestibular neuronitis, cerebrovascular diseases such as cerebral infraction, cerebral hemorrhage and chronic cerebral vascular insufficiency, and blood pressure abnormalities such as hypertension, hypotension and orthostatic hypotension.

Early detection of such abnormalities is important in ensuring safety on health of normal healthy people.

As conventional methods of objectively determining recovery situations of patients by rehabilitation, the physical condition of aged persons and the health of normal healthy people described above, and the like, the following methods are known. That is, a method in which a target (e.g. color marker) is attached to a predetermined site of a subject, and the target is photographed by a television camera to analyze motions of the subject, or the like, is known (e.g. see Japanese Patent Laid-Open No. 06-50983, hereinafter referred to as Patent Document 1).

Furthermore, the entire disclosure of Patent Document 1 are incorporated herein by reference in its entirety.

However, as described above, the time period over which data for analysis of motions for managing the physical conditions of patients after rehabilitation and patients just recovered from illnesses, or controlling an abnormal state of normal healthy people such as giddiness on standing up is limited to the time period over which a manager can take part in analysis of motions of a subj ect, thus making it impossible to check the situation of the subject all the time.

Furthermore, in this case, work for obtaining and processing data of motions of the subject is left to the personal point of view or personal judgment of the manager, and the work requires enormous efforts. Thus, it is generally difficult to evaluate the data obtained in this way as an objective measure.

Furthermore, in the method in which the motions of the subject are analyzed with a television camera as described in Patent Document 1, the situation can be known only within the range of installation of the television camera. Thus, a method enabling the situation to be evaluated all the time and in an objective manner has been required.

Furthermore, when the above management data is objectively evaluated, the manager should manually extract from all obtained data a range of data of a certain behavior to be evaluated. However, this work brings about an enormous burden on the manager.

Furthermore, if a recovery state of a patient under rehabilitation is evaluated periodically over a long period of time, the following problems arise.

That is, evaluation of some data may be skipped inadvertently. Furthermore, since work for extracting data to be evaluated is conducted according to personal judgment of the manager, it cannot be assured that a range of clipped data always meets certain conditions from an objective point of view. Thus, for example, there arises a problem such that only evaluation results of low accuracy including data of other behavior, which should not be essentially included in evaluation, can be obtained.

### Disclosure of the Invention

In view of the problems of conventional techniques described above, the invention of this application has as its object the provision of a body motion evaluation apparatus, a body motion evaluation system and the like capable of evaluating a recovery situation of a motion function and the like of subjects more easily and accurately compared with the conventional techniques.

The first invention is a body motion evaluation apparatus capable of being attached to a human body, comprising:
acceleration detecting means of detecting information about an acceleration produced with a motion of a human body;
calculating means of performing predetermined calculation based on said information about the acceleration detected by said acceleration detecting means;
calculation controlling means of setting a predetermined calculation time period in said calculation by said calculating means; and
signal receiving means of receiving from outside a first behavior detection signal including information about detection of a start or end of a first predetermined behavior of said human body,
wherein said calculation controlling means determines a start point of said calculation based on said first behavior detection signal received by said signal receiving means, and sets as said calculation time period a time period from said start point until predetermined time elapses.

The second invention is a bodymotion evaluation apparatus capable of being attached to a human body, comprising:
acceleration detecting means of detecting information about an acceleration produced with a motion of a human body;
calculating means of performing predetermined calculation based on the information about the acceleration detected by the acceleration detecting means;
calculation controlling means of setting a predetermined calculation time period in the calculating means; and
signal receiving means of receiving from outside a first behavior detection signal including information about detection of a start or end of a first predetermined behavior of the human body, and of receiving from outside a second behavior detection signal including information about detection of a start or end of a second predetermined behavior of the human body,
wherein the calculation controlling means determines a start point of the calculation based on the first behavior detection signal received by the signal receiving means, determines an end point of the calculation based on the second behavior detection signal, and sets as the calculation time period a time period between the start point of the calculation and the end point.

The third invention is the body motion evaluation apparatus according to the first invention, comprising controlling means of controlling said acceleration detecting means, wherein said controlling means turns on the power of said acceleration detecting means at a time when said first behavior detection signal is detected by said signal receiving means, and turns off the power of said acceleration detecting means after predetermined time elapses.

The fourth invention is the body motion evaluation apparatus of the second invention, comprising controlling means of controlling the acceleration detecting means,
wherein the controlling means turns on the power of the acceleration detecting means from a time when the first behavior detection signal is detected by the signal receiving means, and turning off the power of the acceleration detecting means from a time when the second behavior detection signal is detected.

The fifth invention is a body motion evaluation system comprising:
first behavior detecting means of detecting a start or end of said first predetermined behavior of a human body, and outputting said first behavior detection signal; and
the body motion evaluation apparatus according to the first or the second inventions.

The sixth invention is a body motion evaluation system comprising:
first behavior detecting means of detecting a start or end of the first predetermined behavior of a human body and outputting the first behavior detection signal;
second behavior detecting means of detecting a start or end of the second predetermined behavior of a human body and outputting the second behavior detection signal; and
the body motion evaluation apparatus of the second or fourth invention.

The seventh invention is the body motion evaluation system according to the sixth invention, wherein said first behavior detecting means is infrared light projecting means or electric field generating means, and
said signal receiving means is (1) infrared light receiving means or (2) electric field receiving means and signal demodulating means.

The eighth invention is the body motion evaluation system of the sixth invention, wherein at least one of the first behavior detecting means and the second behavior detecting means is infrared light projecting means or electric field generating means, and
the signal receiving means is (1) infrared light receiving means or (2) electric field receiving means and signal demodulating means.

The ninth invention is the body motion evaluation system according to the seventh invention, wherein said firstbehavior detecting means comprises
(1) signal sending means of sending said first behavior detection signal; and
(2) passage detecting means of detecting a passage state in which an object passes through a predetermined detection range, door opening/closing means of detecting open/close of a predetermined door, or load detecting means of detecting a load of an object at a predetermined location.

The tenth invention is the body motion evaluation system of the sixth invention, wherein the first behavior detecting means or the second behavior detecting means comprises:
(1) signal sending means of sending the first or second behavior detection signal; and
(2) passage detecting means of detecting a passage state in which an object passes through a predetermined detection range, door opening/closing means of detecting open/close of a predetermined door, or load detecting means of detecting a load of an object at a predetermined location.

The eleventh invention is the body motion evaluation system of the ninth or tenth invention, wherein the passage detecting means is a floor reaction force sensor within a predetermined detection range or a distance sensor detecting a shield.

The twelfth invention is the bodymotion evaluation system according to the eighth invention, wherein said load detecting means is departure/seating detecting means of detecting a departure state in which an obj ect departs from a toilet seat, or detecting a seating state in which said object is seated on said toilet seat.

The thirteenth invention is the body motion evaluation system according to the ninth invention, wherein said departure/seating detecting means comprises
(1) a load sensor detecting a load applied to said toilet seat;
(2) departure/seating state determining means of determining a departure state from a load detected by said load sensor.

The fourteenth invention is the body motion evaluation system according to the tenth invention, wherein said departure/seating state determining means
(1) makes a determination as said departure state at a time when the load detected by said load sensor is detected as a load equal to or smaller than a first predetermined value, or
(2) makes a determination as said departure state at a time when the load detected by said load sensor is detected as a load equal to or smaller than the first predetermined value, and predetermined time elapses.

The fifteenth invention is the body motion evaluation system according to the eleventh invention, wherein said departure/seating state determining means determines that said object is in a seating sate at a time when it is detected that the load detected by said load sensor is equal to or greater than the first predetermined value.

The sixteenth invention is the body motion evaluation apparatus according to the second invention, wherein said acceleration detecting means detects
(1) a first acceleration being an acceleration in a body axis direction of said human body; and
(2) at least any one of a second acceleration being an acceleration orthogonal to said body axis direction and in a front direction of said human body, and a third acceleration orthogonal to both the body axis direction of said human body and the front direction of the human body.

The seventeenth invention is the body motion evaluation apparatus according to the third invention, wherein said acceleration detecting means detects an acceleration direction of gravity based on said first acceleration and said second acceleration detected.

The eighteenth invention is the body motion evaluation apparatus according to the fourth invention, wherein said calculating means calculates the result of integration of a variation in a signal obtained by said acceleration detecting means, or a period of variation in said signal.

The nineteenth invention is the body motion evaluation system of the fifth or sixth invention, wherein the body motion evaluation apparatus further comprises:
storing means of storing results of calculation by the calculating means; and
displaying means of displaying results of calculation by the calculating means,
wherein the displaying means displays in time sequence the stored results stored in the storing means.

The twentieth invention is the body motion evaluation system of the nineteenth invention, wherein the body motion evaluation apparatus further comprises internal sending means of sending the results of calculation by the calculating means or the stored results stored in the storing means, and
the body motion evaluation system further comprises
(1) external receiving means of receiving the sent results sent by the internal sending means; and
(2) second displaying means of displaying the sent results.

The twenty-first invention is the body motion evaluation system of the twentieth invention, wherein the internal sending means sends contents to be sent when a stored result is newly added in the storing means , or when the calculation is performed by the calculating means.

The twenty-second invention is the body motion evaluation system of the nineteenth invention, further comprising second storing means of storing received results received by the external receiving means.

The twenty-third invention is the body motion evaluation system of the twenty-second invention, further comprising inputting means of selecting any of the stored results stored in the second storing means, and making the second displaying means display the selected result.

The twenty-fourth invention is a program of making a computer function as the calculating means and the calculation controlling means of the body motion evaluation apparatus of the first or second invention.

The twenty-fifth invention is a recording medium carrying the program of the twenty-fourth invention, which can be processed by a computer.

### Brief Description of the Drawings

Figure 1 is a schematic block diagram of a body motion evaluation apparatus according to Embodiment 1 of the present invention;
Figure 2 is a schematic block diagram of the body motion evaluation apparatus according to Embodiment 1 of the present invention;
Figure 3 is a schematic block diagram of the body motion evaluation apparatus according to Embodiment 1 of the present invention;
Figure 4 shows one example of an operational situation according to Embodiment 1 of the present invention;
Figure 5 shows one example of the operational situation according to Embodiment 1 of the present invention;
Figure 6 shows one example of the operational situation according to Embodiment 1 of the present invention;
Figure 7 shows an example of a combination of first behavior detecting means/second behavior detecting means according to Embodiment 1 of the present invention, and a place thereof;
Figure 8 is a schematic diagram showing an example of installation of a distance sensor and a passage sensor;
Figure 9 is a schematic diagram showing a configuration of departure/seating detecting means according to Embodiment 1 of the present invention;
Figures 10(a) and 10(b) are graphs showing a change in load according to Embodiment 1 of the present invention;
Figure 11 is a schematic diagram of the body motion evaluation apparatus in an operation state according to Embodiment 1 of the present invention;
Figures 12 (a) and 12(b) show acceleration waveforms of walking in the gravity direction just after an operation and ten days after the operation according to Embodiment 1 of the present invention;
Figure 13 is a schematic block diagram of the body motion evaluation apparatus according to Embodiment 2 of the present invention;
Figure 14 shows time required for one walk in walking according to Embodiment 2 of the present invention;
Figure 15 is a schematic block diagram of a body motion evaluation system according to Embodiment 3 of the present invention;
Figure 16 shows an overall body motion evaluation system in a hospital;
Figure 17 is a schematic block diagram according to Embodiment 4;
Figure 18 is a block diagram of bed departure detecting means according to Embodiment 4;
Figure 19 is a block diagram of a posture sensor according to Embodiment 4;
Figure 20 is an explanatory view showing evaluation of an equilibrium state according to Embodiment 4;
Figure 21 is a schematic diagram showing evaluation of the equilibrium state according to Embodiment 4;
Figure 22 is a schematic block diagram according to Embodiment 5;
Figure 23 is a schematic block diagram according to Embodiment 6; and
Figures 24(a) to 24(c) are schematic diagrams showing evaluation of the equilibrium state according to Embodiment 4.

### [Description of Symbols]

- 11.: Body motion evaluation apparatus
- 12.: Acceleration detecting means
- 13.: Calculating means
- 14.: Calculation controlling means
- 15.: Displaying means
- 16.: Behavior detecting means
- 17.: Reception port
- 18.: Acceleration power supply
- 21.: First behavior detecting means
- 22.: Second behavior detecting means
- 33.: Acceleration power supply controlling means
- 41.: Load sensor
- 51.: Distance sensor
- 61.: Floor reaction force sensor
- 81.: Load sensor
- 82.: Distance sensor
- 83.: Passage sensor
- 84.: Floor reaction force sensor
- 91.: Load sensor
- 92.: Signal processing sensor
- 93.: Departure/seating detecting means
- 111.: Standing position state
- 112.: Sitting position state
- 113.: Lie position state
- 131.: Storing means
- 151.: Sending means
- 152.: Receiving means
- 153.: Second displaying means
- 161.: Second storing means
- 162.: Inputting means
- 211.: Bed departure detecting means
- 212.: Posture sensor
- 213.: Base station
- 214.: Management terminal
- 221.: Load sensor
- 222.: Signal processing means
- 231.: Acceleration sensor
- 232.: Send controlling unit
- 233.: Sending unit
- 251.: Calculation result range
- 271.: Information sending means
- 272.: Information receiving means
- 281.: Cellular phone

### Best Mode for Carrying Out the Invention

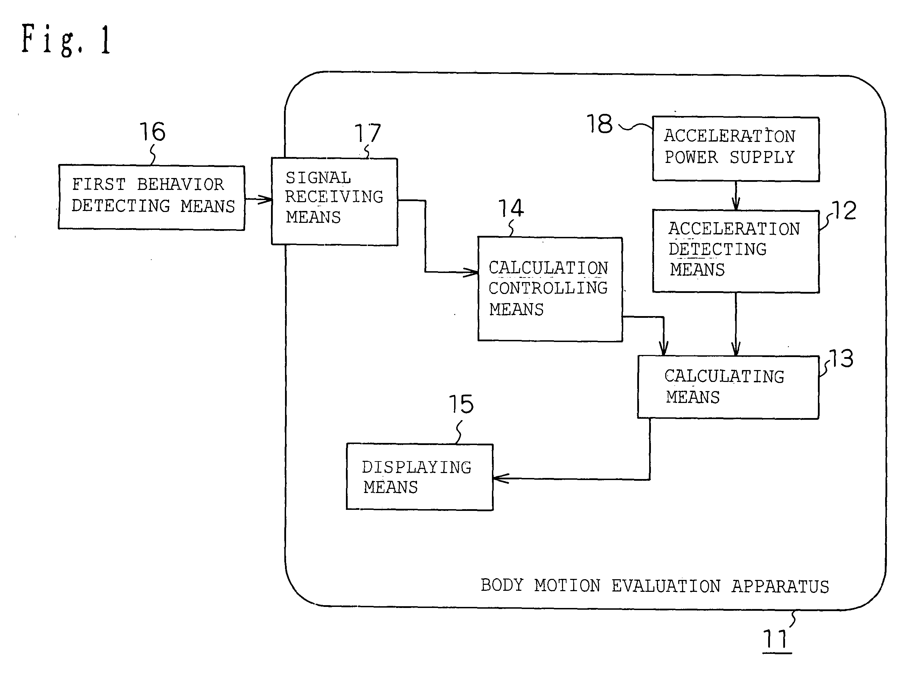

Embodiments of the present invention will be described below using Figures 1 to 16.

### Embodiment 1

A configuration of a body motion evaluation system of Embodiment 1 will be described with reference to the drawings.

Figure 1 is a schematic block diagram showing one example of a body motion evaluation apparatus 11 and the body motion evaluation system of Embodiment 1 of the present invention.

A configuration of the body motion evaluation apparatus 11 in this Embodiment will be described below.

The body motion evaluation apparatus 11 of this Embodiment comprises acceleration detecting means 12 of detecting an acceleration produced with a motion of a human body (subject), calculating means 13 of performing predetermined calculation for the acceleration detected by the acceleration detecting means 12, calculation controlling means 14 of determining a calculation time period in the calculating means 13, and displaying means of displaying results of calculation by the calculating means 13.

Furthermore, the body motion evaluation apparatus 11 is attached to a subject for use.

The calculation controlling means 14 receives a behavior detection signal associated with detection of a start or end of a predetermined behavior of the subject from outside via signal receiving means 17, and defines as a calculation time period a time period from a time when the behavior detection signal is detected until predetermined time elapses. The behavior detection signal is a signal sent from first behavior detecting means 16 installed outside the body motion evaluation apparatus 11.

Furthermore, in the system shown in Figure 1, if the behavior of the subject in walk training using parallel bars is to be evaluated, the calculation time period is set in the following manner.

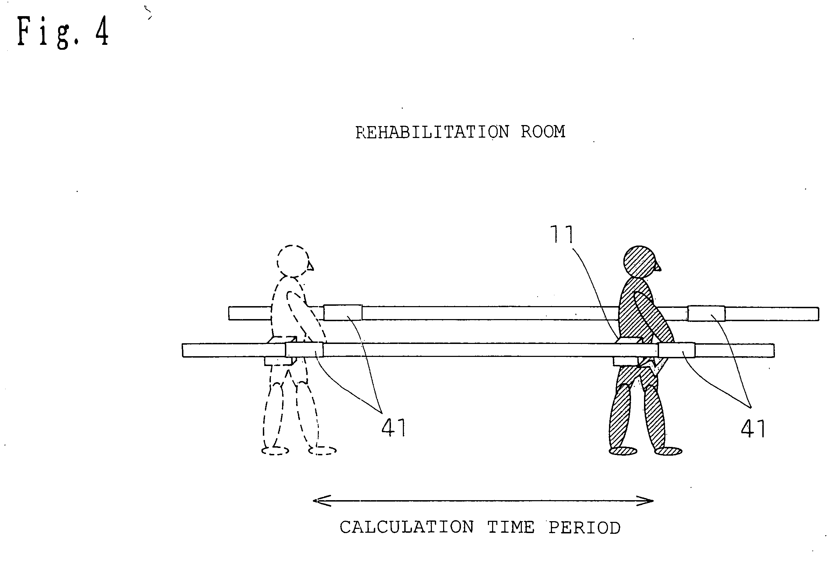

That is, assume that maximum one minute is required for walking from one end to the other of the parallel bar (see Figure 4). In this case, the first behavior detecting means 16 detects the subject has passed through the one end, and the calculation controlling means 14 receives a signal from the first behavior detecting means 16, and then sets as a calculation time period a time period until predetermined 30 minutes elapse, for example.

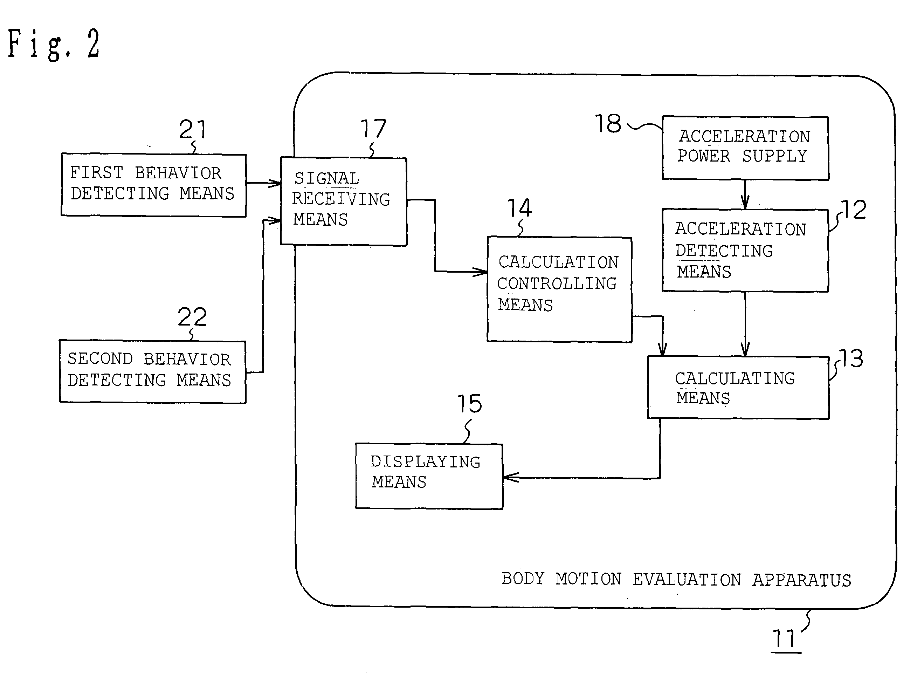

Furthermore, the configuration shown in Figure 2 is another example of the body motion evaluation system of the present invention.

In this case, as shown in Figure 2, the configuration is such that first behavior detecting means 21 of detecting a start or end of a first predetermined behavior of the subject, and second behavior detecting means 22 of detecting a start or end of a second predetermined behavior of the subject are installed outside the body motion evaluation apparatus 11. Consequently, the calculation time period may be defined by making a setting such that calculation is started based on a signal from the first behavior detecting means 21, and calculation is ended based on a signal from the second behavior detecting means 22.

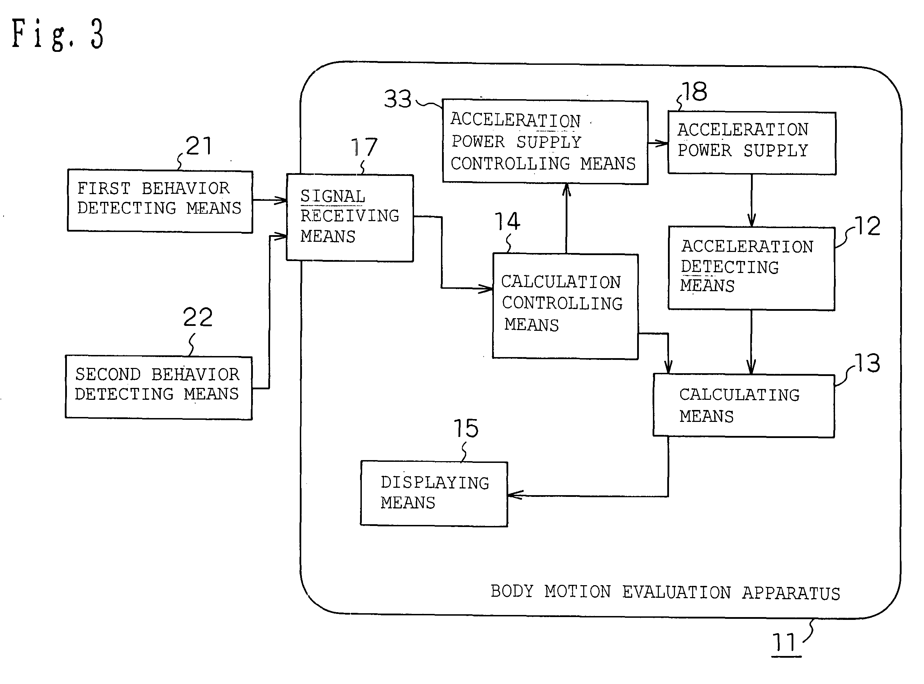

Furthermore, the configuration shown in Figure 3 is still another example of the body motion evaluation system of the present invention.

In this case, as shown in Figure 3, the configuration is such that acceleration power supply controlling means 33 of controlling a power supply 18 of the acceleration detecting means 12 is provided.

Consequently, a configuration may be provided such that the supply of power to the acceleration detecting means 12 is started at a time when the behavior detection signal is detected by the signal receiving means 17, and the supply of power to the acceleration detecting means 12 is stopped after predetermined time elapses.

This configuration makes it possible to restrict power consumptions during time periods other than the evaluation time period.

Figure 4 shows one example of use of the system of Embodiment 1 of the present invention.

Use of the body motion evaluation system in this Embodiment will be described below with reference to Figure 4.

Figure 4 represents a rehabilitation room, and shows a situation in which walk training using parallel bars is conducted as a training menu. According to subject motion evaluation data obtained through walk training using parallel bars, the walk training itself is conducted under invariant unified conditions, thus making it possible to obtain reliable results when a recovery situation of a motion function of the subject are evaluated over a long period of time. In this way, the situation of a behavior made under unified conditions is simply called a unified motion situation.

Therefore, in order that motion evaluations of walk training using parallel bars (hereinafter, referred to as unit walk training) can be conducted highly objectively, accurately and continuously, a configuration enabling automatic extraction of only data for a time period over which the subject walks has very important implications.

Thus, as shown in Figure 4, in this Embodiment, load sensors 41 having first behavior detecting means 21 and second behavior detecting means 22, respectively, are provided on both ends of the parallel bar. Consequently, a calculation time period is set through calculation controlling means 14 by providing behavior detection information sending means of sending timing in which a wearer of the body motion evaluation apparatus 11 pulls his or her weight on the load sensors 41 to the body motion evaluation apparatus 11 as an output of a calculation start/calculation end.

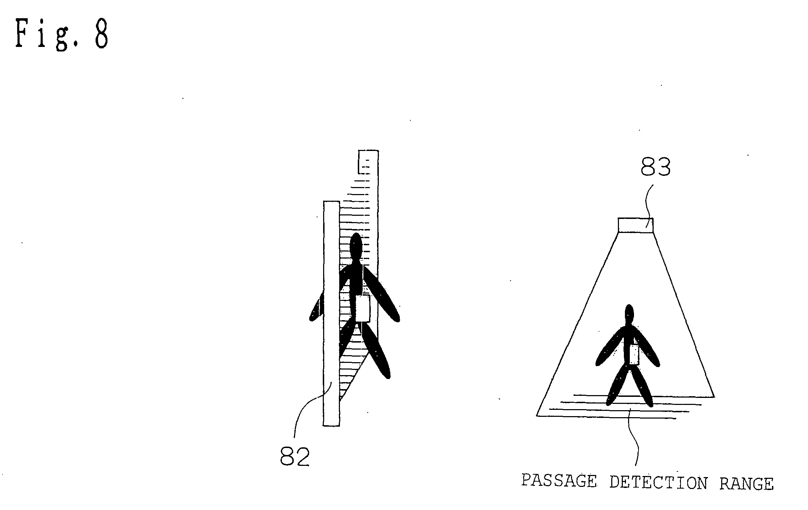

Furthermore, here, existence of the subject may be detected while detecting the movement direction of the subject at the same time by installing a plurality of distance sensors, pyroelectric sensors (see Figure 8) and the like so that their detection areas encompass the detection areas of the load sensors 41.

This configuration makes it possible to detect whether the subject enters or leaves the range of the parallel bar. Consequently, the time period of walk training with the parallel bar can be extracted more accurately.

Furthermore, as communication of the behavior detection signal between the load sensor 41 and the body motion evaluation apparatus 11, wireless communication can be considered.

However, for avoiding a situation in which the apparatus 11 of a wearer other than the wearer of the body motion evaluation apparatus 11 actually conducting walk training with the parallel bar erroneously detects the wireless communication, an apparatus of which the range of wireless communication is about several tens centimeters so that only moderate range transmission can be performed is preferably used.

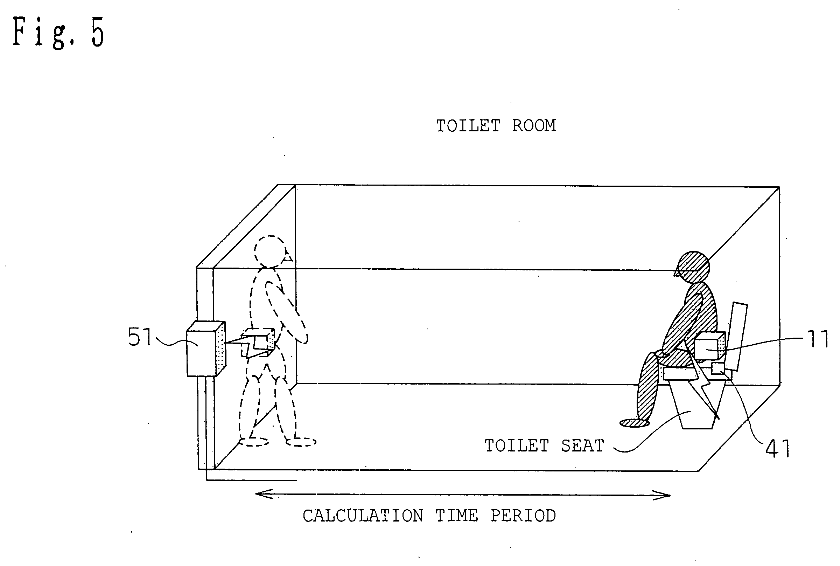

Furthermore, Figure 5 shows one example of use of the system of Embodiment 1 of the present invention.

Figure 5 represents a behavior beginning with entrance into a toilet room and ending with seating on a toilet seat.

This behavior is a behavior conducted under relatively invariant unified conditions in every day life, and is therefore suitably used as a unified motion situation in carrying out motion evaluations of the subject over a long period of time, thus making it possible to obtain highly reliable results.

Thus, a distance sensor 51 detecting passage of the subject is provided as first behavior detecting means 21 at the entrance of the toilet room, the load sensor 41 is provided as second behavior detecting means 22 on the toilet seat, and detection timing in the sensors is sent to the body motion evaluation apparatus 11 as an output of a calculation start/calculation end to set a calculation time period through the calculation controlling means 14.

Furthermore, here, the distance sensor 51 detecting passage of the subject is provided as the first behavior detecting means 21, but this may be a floor reaction force sensor or the like.

Furthermore, instead of the distance sensor 51, a passage detection sensor such that a plurality of pyroelectric sensors (see Figure 8) are used to detect a passage direction may be used.

In this way, whether the subject enters or leaves the toilet room can be detected while detecting the direction of movement at the same time.

Thus, time periods during entrance into and leaving from the toilet room excluding a time period over which the subj ect is seated on the toilet seat can be extracted more accurately.

Furthermore, as in the case of Figure 4, as communication of the behavior detection signal between the distance sensor 41 and load sensor 51 and the body motion evaluation apparatus 11, wireless communication can be considered.

In this case, however, as described above, for avoiding erroneous detection by the apparatus 11 of a wearer other than the wearer of the body motion evaluation apparatus 11 actually involved in a toilet room entrance behavior, an apparatus of which the range of wireless communication is about several tens centimeters so that only moderate range transmission can be performed is preferably used.

Furthermore, by providing means of detecting a time such as a clock, data of a usual toilet use behavior can be separated from data of an unexpected toilet use behavior to detect a toilet room entrance behavior and leaving behavior as a unified behavior situation.

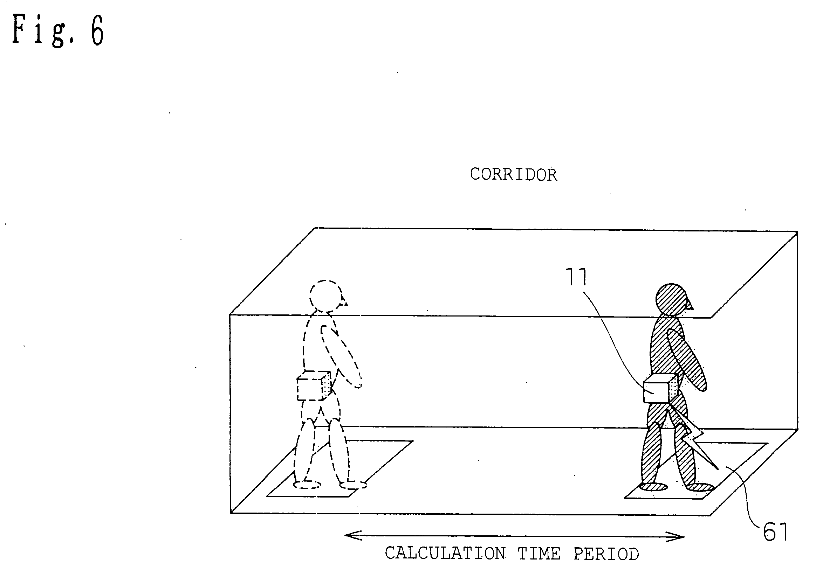

Furthermore, Figure 6 shows one example of use of the system of Embodiment 1 of the present invention.

Figure 6 represents a behavior at the time of passage through a corridor.

Floor reaction force sensors 61 detecting passage of the subject, having functions of first behavior detecting means 21 and second behavior detecting means 22 are provided at two locations in the corridor.

Consequently, detection timing in the sensors is sent to the body motion evaluation apparatus 11 as an output of a calculation start/calculation end to set a calculation time period through the calculation controlling means 14.

Furthermore, here, the case is described where the floor reaction force sensor 61 detecting passage of the subject is provided as first behavior detecting means 21 and second behavior detecting means 22, but the invention is not limited thereto, and the distance sensor 51 may be provided.

Furthermore, a passage detection sensor such that a plurality of pyroelectric sensors are used to detect a passage direction may be used instead of the floor reaction force sensor 61.

In this way, the direction of movement along the corridor can be detected while detecting the direction of movement at the same time, thus making it possible to extract a unified behavior situation more accurately.

Furthermore, as in the case of Figure 4, as communication of the behavior detection signal between the distance sensor 51 and load sensor 41 and the body motion evaluation apparatus 11, wireless communication can be considered.

In this case, however, as described above, for avoiding erroneous detection by the apparatus 11 of a wearer other than the wearer of the body motion evaluation apparatus 11 actually involved in a toilet room entrance behavior, an apparatus of which the range of wireless communication is about several tens centimeters so that only moderate range transmission can be performed is preferably used.

Furthermore, if a hand rail is provided along the corridor, and it is previously known that the wearer of the body motion evaluation apparatus 11 needs the help of the hand rail when he or she walks, the load sensor may be mounted on the hand rail as in the case of Figure 4.

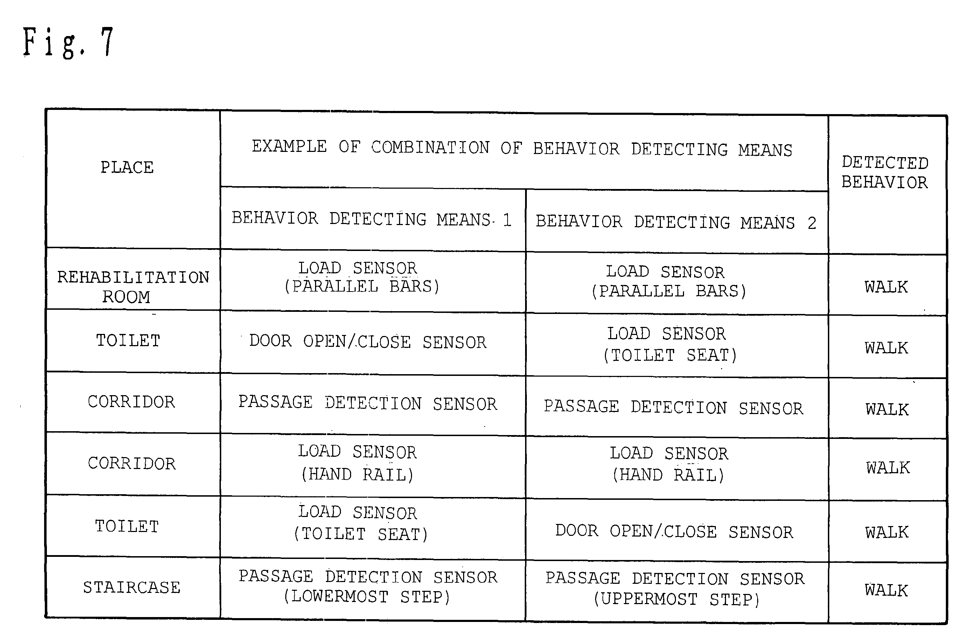

Furthermore, Figure 7 shows an example of a combination of first behavior detecting means 21 and second behavior detecting means 22 as shown in Figures 4 to 6, and places where they are installed.

Specific examples of the passage detection sensor installed on the corridor, shown in Figure 7, include, for example, a distance sensor 82 and an infrared sensor as one example of a passage sensor 83 as shown in Figure 8, in addition to the floor reaction force sensor 61 and the distance sensor 51. Furthermore, below the names of various kinds of sensors shown in Figure 7, their specific installation places are written in parentheses.

Furthermore, aside from the combination of the sensors described above, a door open/close sensor detecting entrance into a toilet or the like, etc. may be used.

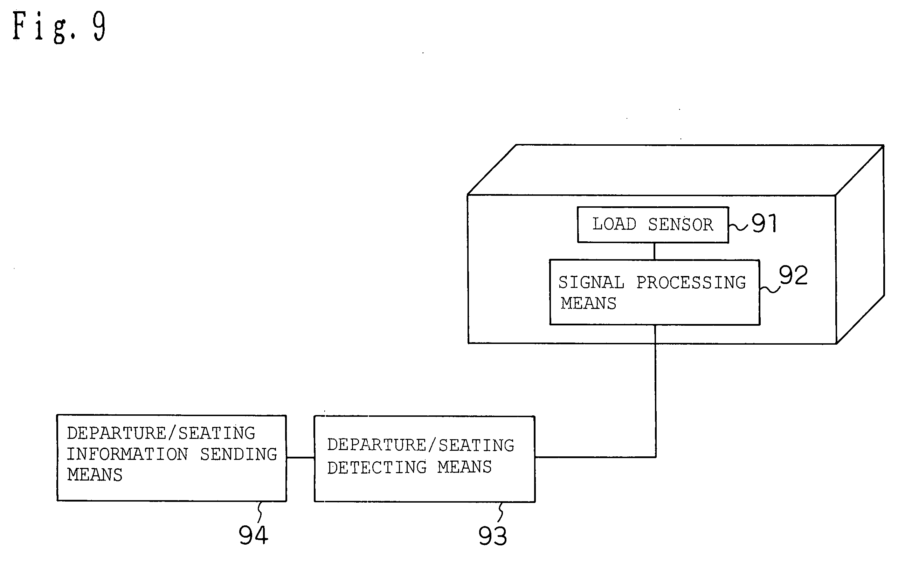

Furthermore, Figure 9 shows a configuration of departure/seating detecting means 93 of one Embodiment of the present invention.

The departure/seating detecting means 91 comprises a load sensor 91 and signal processing means 92, and is installed on a toilet seat to detect a load applied to the toilet seat. The load applied to the load sensor 91 is signal-processed by the signal processing means 92, and a signal corresponding to the load applied to the load sensor 91 is outputted from the signal processing means 92 as a sequential signal. The departure/seating detecting means 93 detects a start of a departure state based on the signal outputted from the signal processing means 92.

Operations of the body motion evaluation system of Embodiment 1 will now be described.

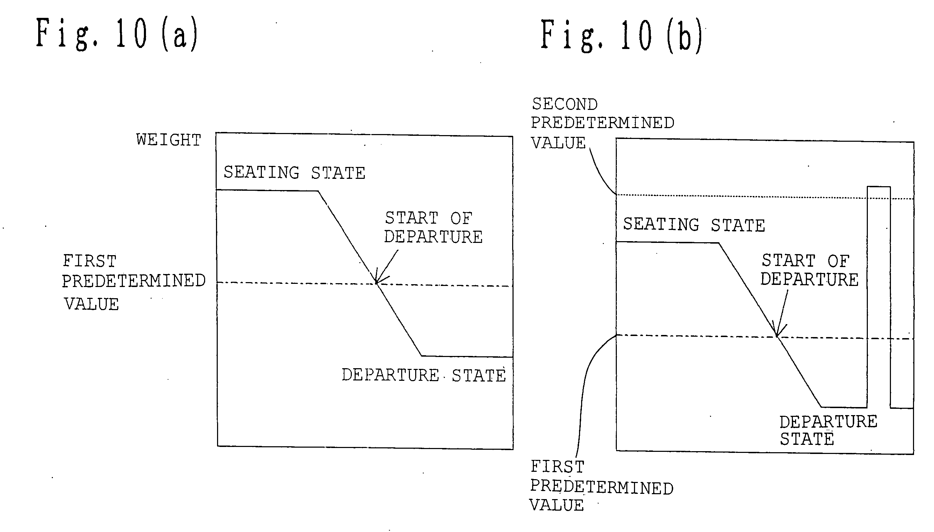

When the subject is seated on the toilet seat, the load sensor 91 detects a load in a seating state shown in Figure 10(a). When the subject relieves himself and departs from the toilet seat, the load detected by the load sensor 91 decreases, and when the subject completely departs from the toilet seat, the load sensor 91 detects a load in a departure state shown in Figure 10(a).

A first predetermined value is set between the load in the seating state and the load in the departure state, and when the load on the load sensor 91 decreases to below the first predetermined value, the departure/seating detecting means 93 detects a start of a departure state of the subject 2.

Furthermore, the subject 2 may suffer an abnormal motion and fall toward the toilet seat within predetermined time after the start of the departure state. In this case, a considerable load exceeding the load in the seating state is applied to the toilet seat as shown in Figure 10(b).

A second predetermined value is set as a threshold of such a load. If the load sensor 91 detects a load equal to or greater than the second predetermined value within predetermined time after the start of the departure state of the subj ect 2, determination as a departure state is cancelled based on the detection.

As described above, the second predetermined value is set, and a distinction is made between the case where the load sensor 91 continuously detects a load equal to or less than the first predetermined value from the start of the departure state of the subject until predetermined time elapses, and the case where the load sensor 91 detects a load equal to or greater than the second predetermined value, whereby a unified behavior situation can be extracted, and a rehabilitation process can be observed more accurately.

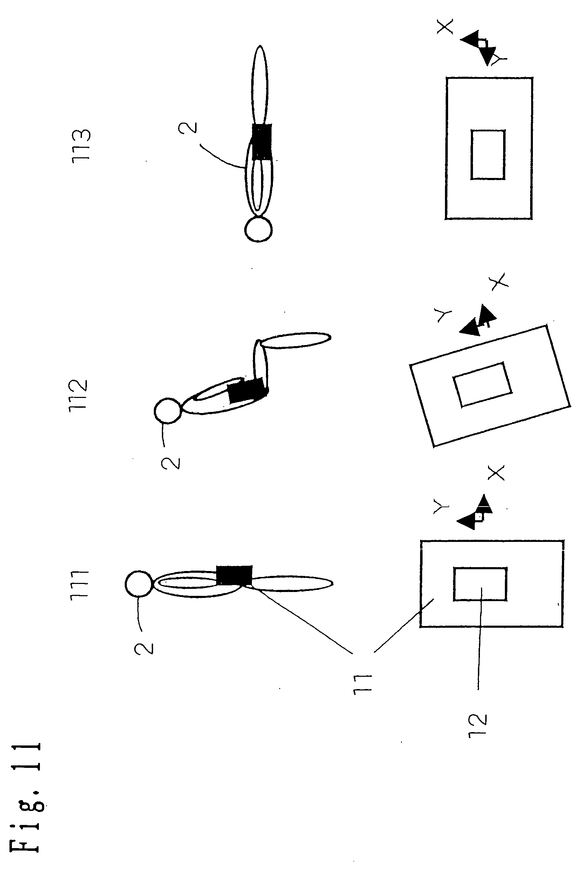

Figure 11 is a schematic diagram of the body motion evaluation apparatus 11 in an operation state.

Furthermore, acceleration detecting means 12 installed in the body motion evaluation apparatus 11 represents biaxial direction-type acceleration detecting means 12 of sensitivity having directivity in a body axis direction of a human body of the present invention and a direction orthogonal to the body axis direction of a human body of the present invention. Furthermore, for the acceleration detecting means 12, a type capable of detecting the gravity direction, such as a capacitance type, is used.

The body motion evaluation apparatus 11 provided with the acceleration detecting means 12 is assumed to be attached to a lumber part of the subject, and a signal proportional to the magnitude of a gravity acceleration changing with the inclination of the acceleration detecting means 12 is sent as an output from the body motion evaluation apparatus 11.

Figure 11 shows states of a standing position 111, a sitting position 112 and a lie position 113 in order from the left. Here, the body axis direction of the human body of the present invention is made to match the y-axis direction as one example, and the direction orthogonal to the body axis direction of the human body of the present invention is made to match the x-axis direction as one example.

In the case of the standing position 111, the gravity direction matches the y-axis, and the front of the subject matches the x-axis. In the case of the sitting position 112 and the lie position 113, the sum of gravity direction components of a first acceleration detected in the x-axis direction and a second acceleration detected in the y-axis direction equals a gravity acceleration value.

Furthermore, if the subject 2 makes a transition from the sitting position 112 to the lie position 113, for example, gravity direction components of the first and second accelerations produced in the x-axis direction and the y-axis direction, respectively (calculated from an angle formed by the x-axis direction or y-axis direction and the gravity direction) are summed into a gravity acceleration value.

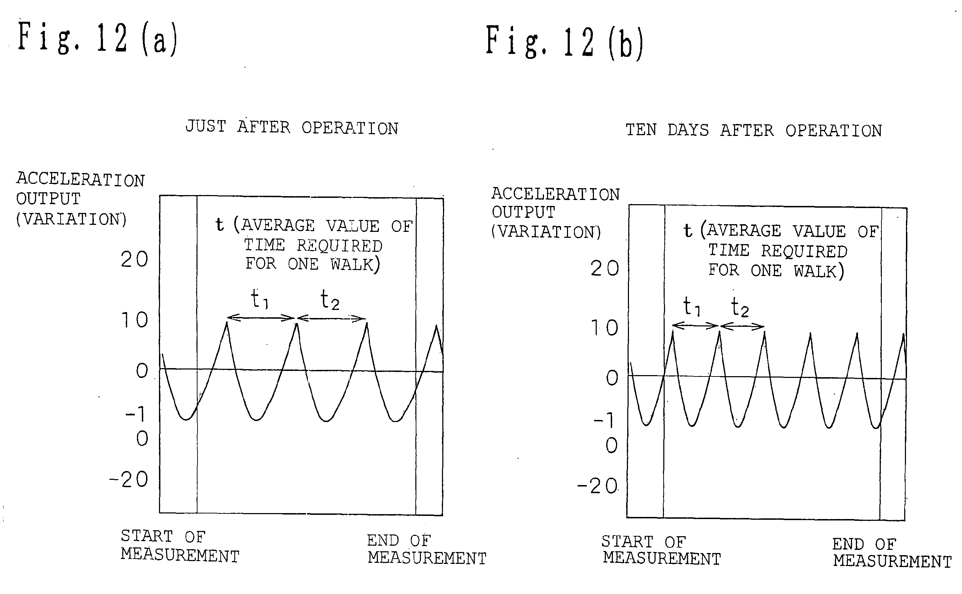

Figures 12 (a) shows an acceleration waveform of walking in the gravity direction just after an operation, and Figure 12 (b) shows an acceleration waveform of walking in the gravity direction ten days after the operation. Calculating means 13 calculates an average value t from time t₁, t₂ ... each required for one walk, and displaying means 15 displays the calculation result. Consequently, the progress of rehabilitation can be acquired as an objective value.

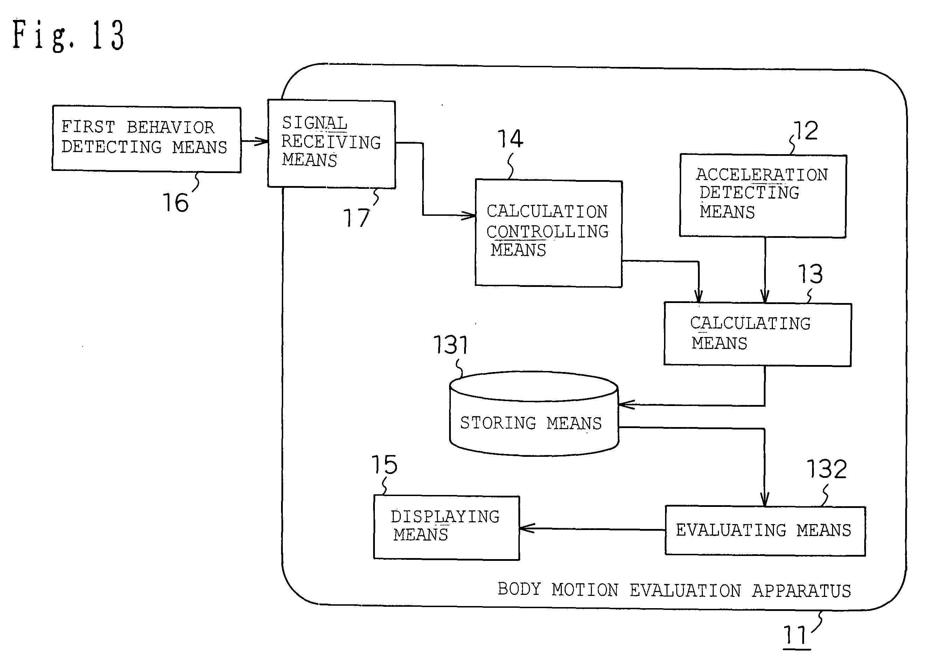

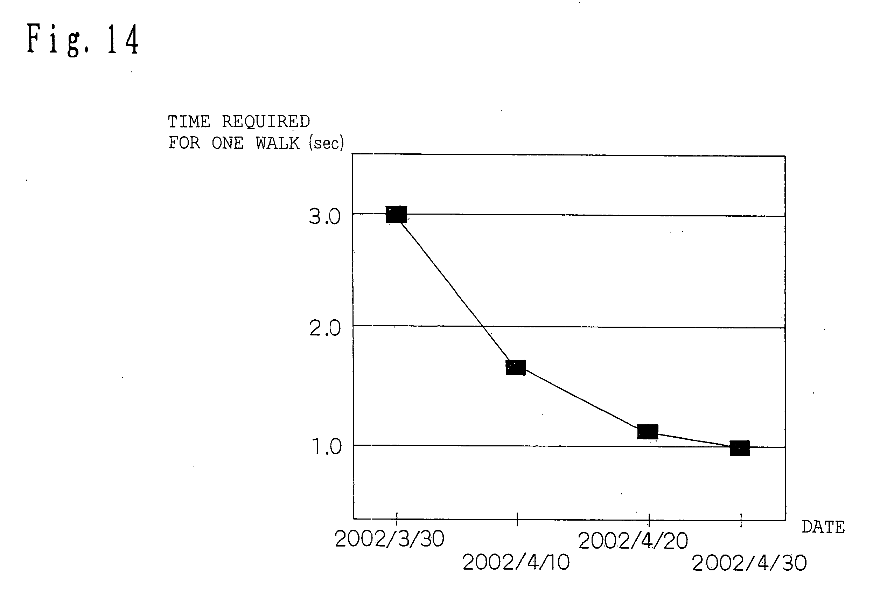

Furthermore, an example of display by the marking means 15 will be further described with reference to Figures 13 and 14 described later.

### Embodiment 2

A configuration of motion evaluation apparatus of Embodiment 2 will be described with reference to the drawings. Figure 13 is a schematic block diagram of the body motion evaluation apparatus of Embodiment 2 of the present invention.

The configuration of the body motion evaluation apparatus in this Embodiment will be described below.

The motion evaluation apparatus of this Embodiment further comprises storing means 131 of storing calculation results in the calculating means 13 and evaluating means 132 of evaluating with time the evaluation results stored by the storing means 131, in addition to the configuration in the body motion evaluation apparatus 11 of Figure 1.

This configuration enables an evaluation result to be compared with pervious one in a relative manner, thus making it possible to observe the advance of rehabilitation in time sequence.

Furthermore, Figure 14 shows time required for one walk when a patient wearing the body motion evaluation apparatus 11, who has undergone a hip joint operation, actually walks. From the graph, it can be understood that time required for the walk of the patient decreases and the condition of the patient improves as days go by after the operation.

### Embodiment 3

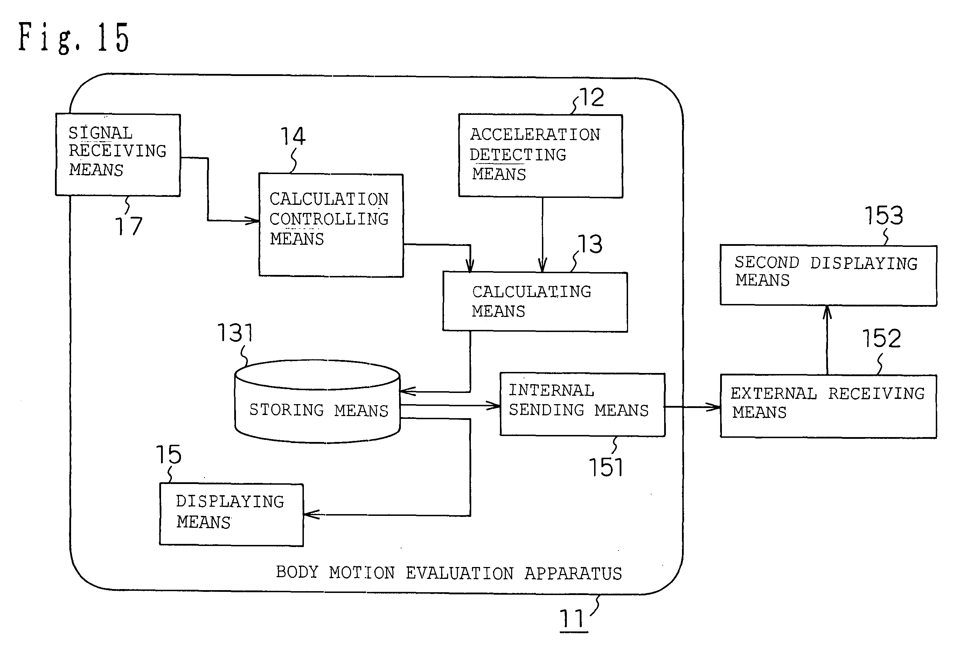

The body motion evaluation system of Embodiment 3 will be described with reference to the drawings. Figure 15 is a schematic block diagram of the body motion evaluation system of Embodiment 3 of the present invention.

The configuration in this Embodiment will be described below.

This Embodiment has a system configuration such that with the body motion evaluation apparatus shown in Figure 13 as a base, internal sending means 151 is further provided in the body motion evaluation apparatus 11, and external receiving means 152 and second displaying means 153 are provided outside the body motion evaluation apparatus 11. Components same as those of Embodiment 2 are given like reference symbols, and descriptions thereof are not presented.

The body motion evaluation apparatus 11 sends calculation results in the calculating means 13 or evaluation results stored by the storing means to the receiving means 152 by the sending means 151.

The receiving means 152 receives information indicating the motion, and displays the same on the second displaying means 153, whereby a doctor in a remote area can know a situation via the second displaying means 153 and for example, the doctor can know the advance of rehabilitation at home.

Furthermore, here, the information may be sent to a family or relative in a remote area, and can be known in the remote area.

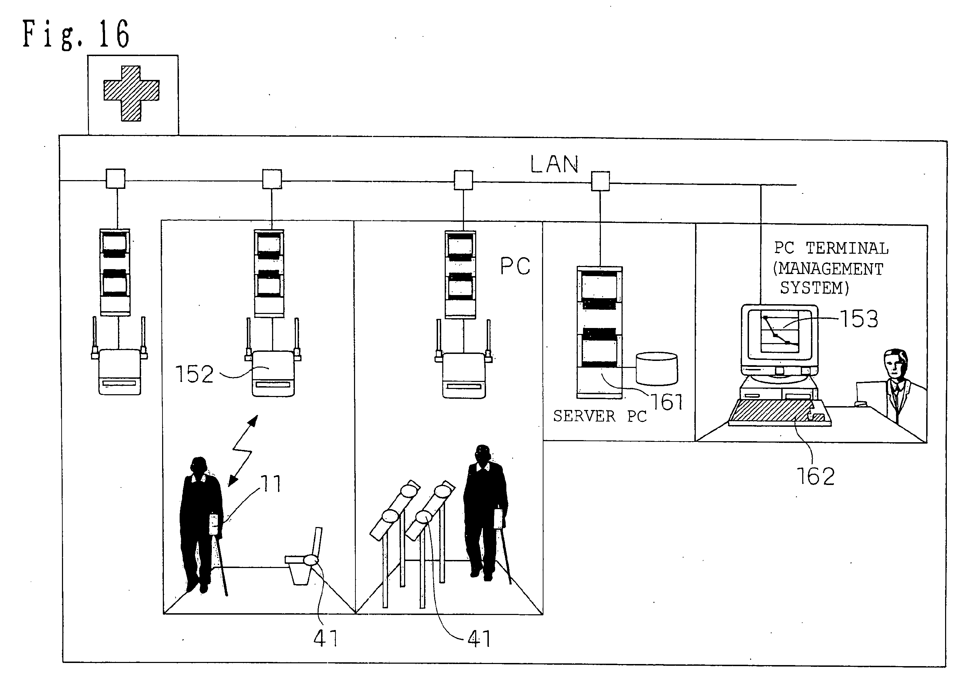

Furthermore, Figure 16 shows an overall body motion evaluation system in a hospital. As shown in Figure 16, second storing means 161 is further provided, and the second displaying means 153 and inputting means 162 are provided in a PC terminal, so that the advance of rehabilitation for each patient can be known at any time.

Furthermore, a program of the present invention is, for example, a program of making a computer perform a function of calculating means 13 of performing predetermined calculation based on information about an acceleration detected by acceleration detecting means 12 (see Figure 1) of detecting information about the acceleration produced with a motion of a subject, and calculation controlling means 14 of setting a predetermined calculation time period in the calculation by the calculating means 13, the program operating in cooperation with the computer.

Here, a start point of the calculation is determined based on a first behavior detection signal received by signal receiving means 17, and the calculation time period is set as a time period from the start point until predetermined time elapses (see Figure 1). Alternatively, a start point of the calculation is determined based on the first behavior detection signal sent from first behavior detecting means 21, which has been received by the signal receiving means 17, an end point of the calculation is determined based on a second behavior detection signal sent from second behavior detecting means 22, and the calculation time period is set as a time period between the start point and the end point of the calculation (see Figures 2 and 3).

The program according to the present invention is, for example, a program of making a computer perform a function of entire or partial apparatus as the body motion evaluation apparatus 11, which evaluates an acceleration from the start of the departure state until predetermined time elapses from signals obtained from the acceleration detecting means 12 and departure/seating detecting means 93, in the body motion evaluation system of the present invention, the program operating in cooperation with the computer.

A medium according to the present invention is, for example, a medium carrying a program of making a computer perform a function of entire or partial apparatus as the body motion evaluation apparatus 11, which evaluates an acceleration from the start of the departure state until predetermined time elapses from signals obtained from the acceleration detecting means 12 and departure/seating detecting means 93, in the body motion evaluation system of the present invention, wherein the medium is computer-readable, and the program read performs the functions in cooperation with the computer.

Furthermore, the "partial apparatus" refers to partial means of one apparatus or partial functions of one means.

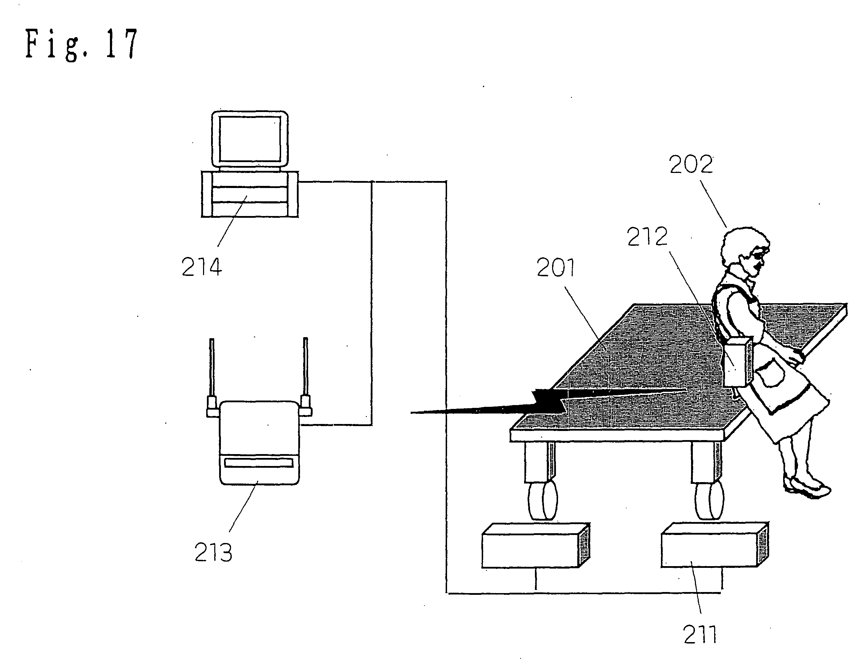

Embodiments of the invention related to the present invention described above will now be described with reference to Figures 17 to 24.

### Embodiment 4

A configuration of a body equilibrium state evaluation system of Embodiment 4 will be described with reference to the drawings.

Figure 17 is a schematic block diagram of the body equilibrium state evaluation system of Embodiment 4 of the present invention.

The configuration of the body equilibrium state evaluation system in this Embodiment will described below.

The body equilibrium state evaluation system of this Embodiment is comprised of bed departure detecting means 211, a posture sensor 212 as one example of acceleration detecting means of the present invention, a base station 213, and a management terminal 214 as one example of a body equilibrium state evaluation/management apparatus of the present invention.

The bed departure detecting means 211, the base station 213 and the management terminal 214 are connected together. In this description, the connection is wired connection, but wireless connection may be employed.

The posture sensor 212 is attached to the body of a subject 202 or held by the subject 202. A specific posture sensor number may be assigned for each posture sensor 212. Information sent from the posture sensor 212 to the base station 213 is sent to the management terminal 214, and information is also sent from the bed departure detecting means 211 to the management terminal 214.

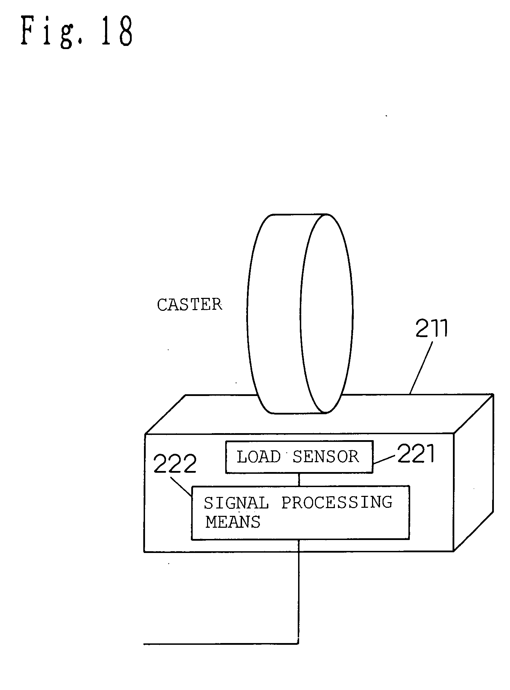

Furthermore, Figure 18 shows a configuration of bed departure detecting means 211 of one Embodiment of the present invention. The bed departure detecting means 211 comprises a load sensor 221 and signal processing means 222, and is installed below a caster of a bed 201 to detect a load applied to the bed 201.

The load applied to the load sensor 221 is signal-processed by the signal processing means 222, and outputs a signal corresponding to the load applied to the load sensor 221 from the signal processing means 222 as a sequential signal. The bed departure detecting means 211 detects a start of a bed departure state based on the signal outputted from the signal processing means 222.

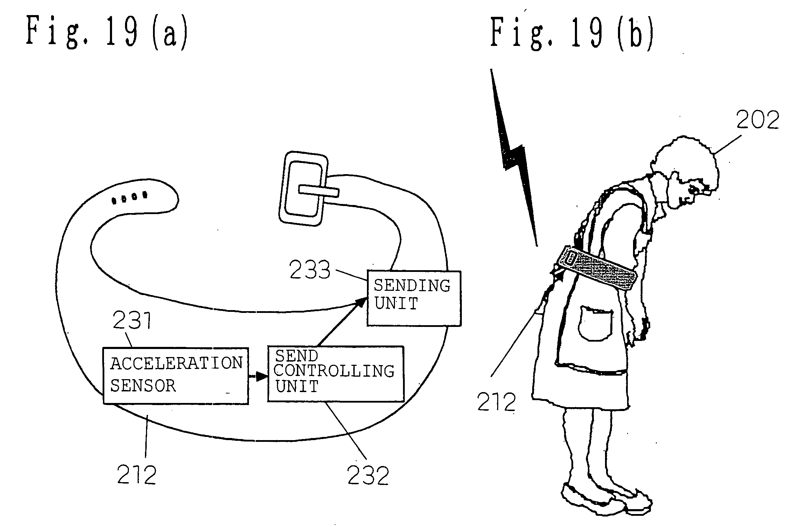

Figure 19(a) is a schematic diagram of the posture sensor 212 detecting a change in posture using an acceleration sensor 231, and Figure 19(b) shows the posture sensor 212 being worn by the subject 202.

The posture sensor 212 is comprised of the acceleration sensor 231 capable of detecting a gravity component, a send controlling unit 232 and a sending unit 233. The acceleration sensor 231 detects an acceleration with a change in posture of a body, and sends the acquired acceleration from the sending unit 233 by wireless.

Furthermore, control of the sending unit 233 is performed by the send controlling unit 232. Here, wireless communication is used for sending, but wired communication may be used.

Operations of the body equilibrium state evaluation system of Embodiment 4 will now be described.

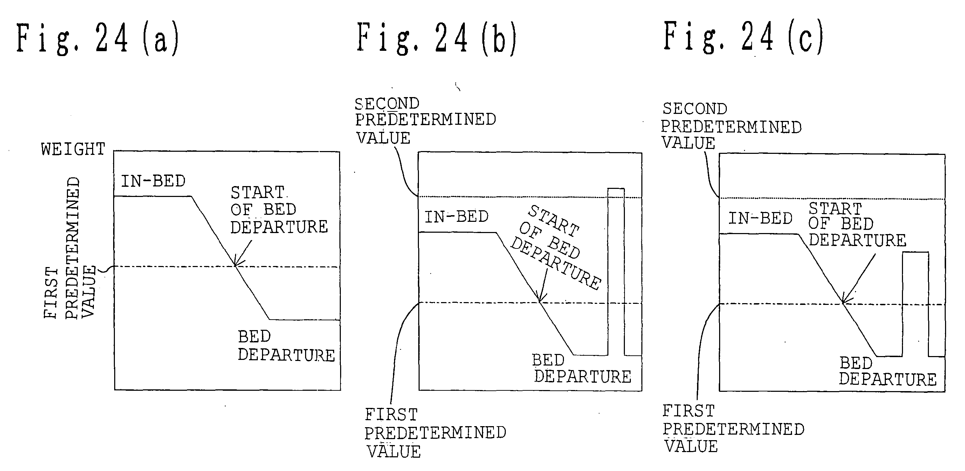

When the subject 202 lies on the bed 201, the load sensor 221 detects a load in an in-bed state shown in Figure 24(a). The load in the in-bed state equals, for example, the load of the bed 201 added to the body weight of the subject.

When the subject 202 gets up and departs from the bed 201, the load detected by the load sensor 221 decreases, and when the subject 202 rises and completely departs from the bed 201, the load sensor 221 detects a load in the bed departure state shown in Figure 24 (a) (e.g. load of the bed 201 alone).

A first predetermined value is set between the load in the in-bed state and the load in the bed departure state, and when the load on the load sensor 221 decreases to below the first predetermined value, the bed departure detecting means 211 detects a start of the bed departure state of the subject 202.

An acceleration of the subject from a start point of the bed departure state until predetermined time elapses is detected by the posture sensor 212, and information of the detected acceleration is sent to the management terminal 214 through the base station 213.

The management terminal 214 stores the information of the acceleration obtained with the posture sensor 212 in a storage apparatus (not shown).

An example of the predetermined time is most preferably 2 to 5 seconds after the bed departure state, for example. If some abnormality of the body equilibrium state such as giddiness on standing up occurs during the predetermined time when the subject 202 departs from the bed, the information of the acceleration obtained with the posture sensor 212 is recorded in the storage apparatus of the management terminal 214.

For description about an operation state of the posture sensor 212, the description about the body motion evaluation apparatus 11, the acceleration detecting means 12 and the like previously described using Figure 11 may be used directly by replacement of terms described below.

Thus, here, overlaps of description are avoided. The replacement of terms is such that the body evaluation apparatus 11 is replaced with the posture sensor 212, the acceleration detecting means 12 is replaced with the acceleration sensor 231, and the subject 2 is replaced with the subject 202. However, the acceleration sensor 231 can detect the gravity direction by means of a weight (not shown) or the like.

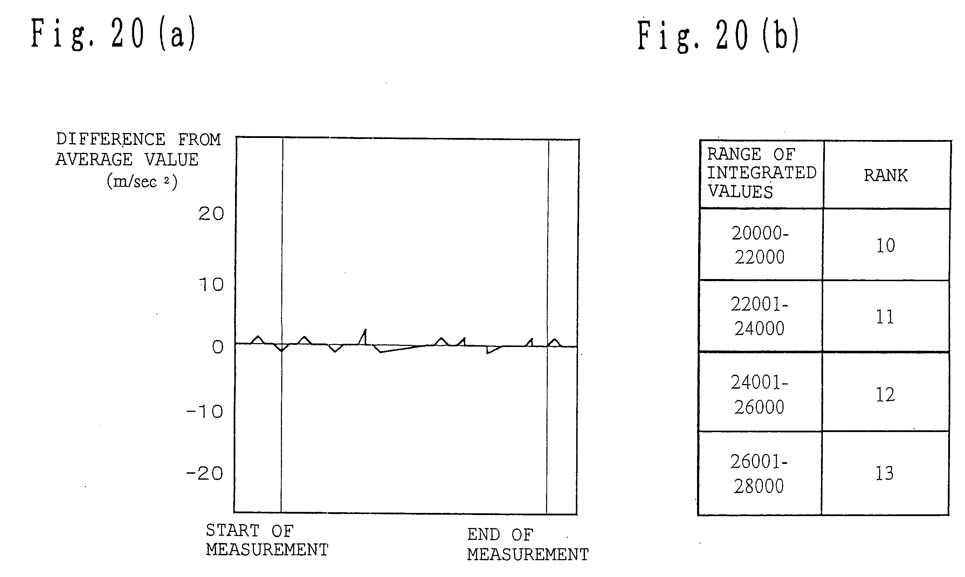

Figures 20(a) and 20(b) show an example of a template for calculation of an equilibrium state of a body in the management terminal 214.

Figure 20(a) shows a difference from an average value of an output (gravity acceleration value) from the posture sensor 212 described with replacement of predetermined terms using Figure 11 in place as described above. This average value is an average value of a gravity acceleration outputted from the posture sensor 212 during a time period between the start and end of measurement (e.g. 5 seconds), and is shown as zero level at the center in the figure.

Figure 20(b) shows a table of classifying, in a ranked order, values obtained by integrating gravity acceleration values outputted from the posture sensor 212 for a predetermined time (e.g. 5 seconds).

The table shown in Figure 20 (b) is such that ranking is made for each group in which a range of results of integration of conceivable gravity accelerations is predefined, and the table is stored in the management terminal 214.

For example, if values shown in Figure 20 (a) are obtained as measurement values for a subj ect, and a 5-second integrated value thereof equals 2340, the value is ranked as 11. This integration processing and ranking processing are performed by the management terminal 214.

Furthermore, Figure 20(b) shows part of the table.

Furthermore, in this description, the posture sensor 212 outputs a sampling value of the gravity acceleration, but the invention is not limited thereto and for example, an integrated value within a predetermined time period may be outputted.

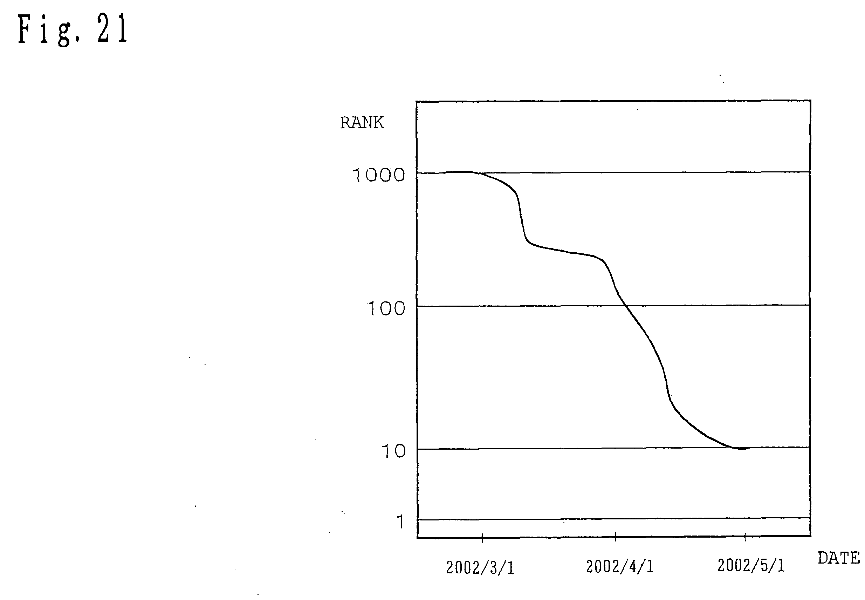

Furthermore, Figure 21 is a schematic diagram showing a chronological transition of a rank obtained by processing in the management terminal 214. As an example, a user under rehabilitation for the leg continuously uses the system over a long period of time and for example, acquires data like those described above when rising in every morning.

As shown in Figure 21, it can be found that the calculation rank becomes small (the integrated value of the acceleration in predetermined time becomes small) as days go by. The fact that the calculation rank becomes small indicates that staggers at the time of bed departure are reduced, i.e. the affected part successfully recovers.

By displaying this process on a display (not shown), for example, or the like, the patient, the doctor and the like can know the situation.

That is, if data of accelerations at the time when the subject 202 departs from the bed is observed with time as described above, the recovery process of the subject 202 can be objectively evaluated.

Furthermore, in the above description, it is assumed that the subject 202 departs from the bed 201 at a time at the time of bed departure, but the case may also be considered where, for example, the subject 202 once starts to depart from the bed 201, and then unconsciously or on its own will returns to the bed 201 again not because some abnormality occurs in the equilibrium state of the body.

When the subject 202 sits down on the bed again, for example, after departing from the bed, the load sensor 221 detects a load again after the subject 202 departs from the bed as shown in Figure 24(c). The load at this time is a load equal to or smaller than the load at the time when the subject 202 is in the bed.

If detecting this state, the management terminal 214 stops capturing the data into the storage apparatus of the management terminal 214 and deletes the data already captured even if the bed departure state is started and measurement of accelerations of the subject 202 is started. Again, the load sensor 221 continuously detects the load equal to or smaller than the first predetermined value for a predetermined time after the subject 202 starts the bed departure state again, and thereafter the data obtained in this way is stored in the storage apparatus of the management terminal 214.

Furthermore, the subject 202 may suffer some abnormality in the equilibrium state and fall toward the bed 201 within predetermined time after the bed departure state is started. In this case, a considerable load exceeding the load at the time when the subject is in the bed is applied to the bed as shown in Figure 24(b).

A second predetermined value is set as a threshold of this load. If the load sensor 221 detects a load equal to or greater than the second predetermined value within predetermined time after the subject 202 starts bed departure, the management terminal 214 determines that such data should be acquired, and the data is stored in the storage apparatus of the management terminal 214.

If the second predetermined value is set as described above, and the load sensor 221 detects continuously a load equal to or smaller than the first predetermined value, or detects a load equal to or greater than the second predetermined value during a time interval from the start of the bed departure state of the subject 202 until predetermined time elapses, the management terminal 214 evaluates an acceleration obtained from the posture sensor 212. Consequently, the doctor, family or the like of the subject 202 can observe a more correct abnormality in the body equilibrium state, and observe the recovery process of the subject 202 more correctly.

### Embodiment 5

The body equilibrium state evaluation system of Embodiment 5 will be described with reference to the drawings.

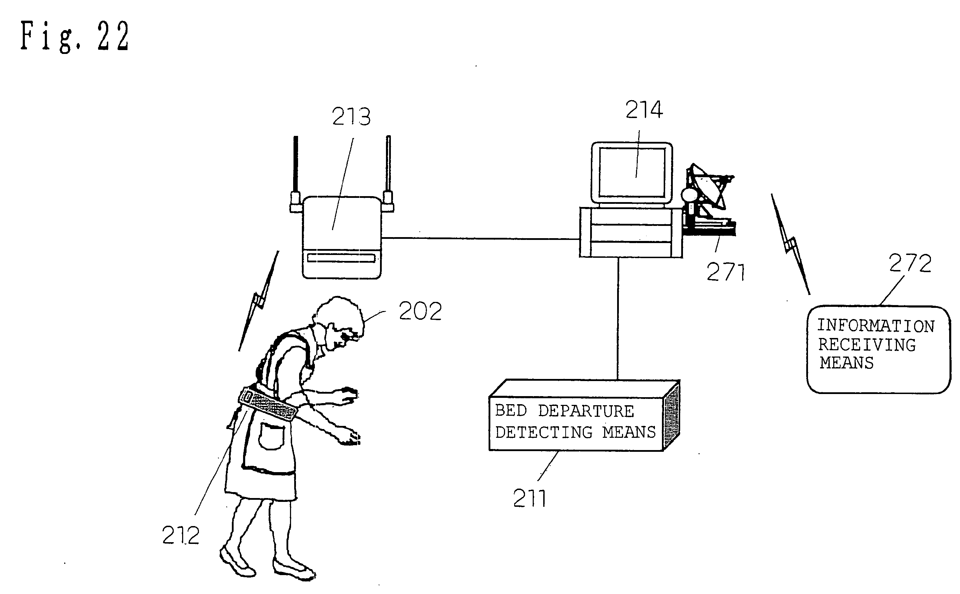

Figure 22 is a schematic block diagram of the body equilibrium state evaluation system of Embodiment 5 of the present invention.

The configuration in this Embodiment will be described below.

This Embodiment has a system configuration such that with the body equilibrium state evaluation system shown in Figure 17 as a base, information sending means 271 is further provided in the management terminal 214, and information receiving means 272 is further provided at a location remote from the management terminal 214. Components same as those of Embodiment 4 are given like reference symbols, and descriptions thereof are not presented.

The posture sensor 212 acquires and collects acceleration information of the subject 202, and calculates a measure indicating the body equilibrium state as in the case of Figure 17. The calculated information indicating the equilibrium state is sent to the information receiving means 272 by the information sending means 271.

The information receiving means 272 receives this information indicating the equilibrium state, whereby the family or doctor in a remote area can know a situation, and can know the advance of rehabilitation, for example.

### Embodiment 6

The body equilibrium state evaluation system of Embodiment 6 of the present invention will be described with reference to the drawings.

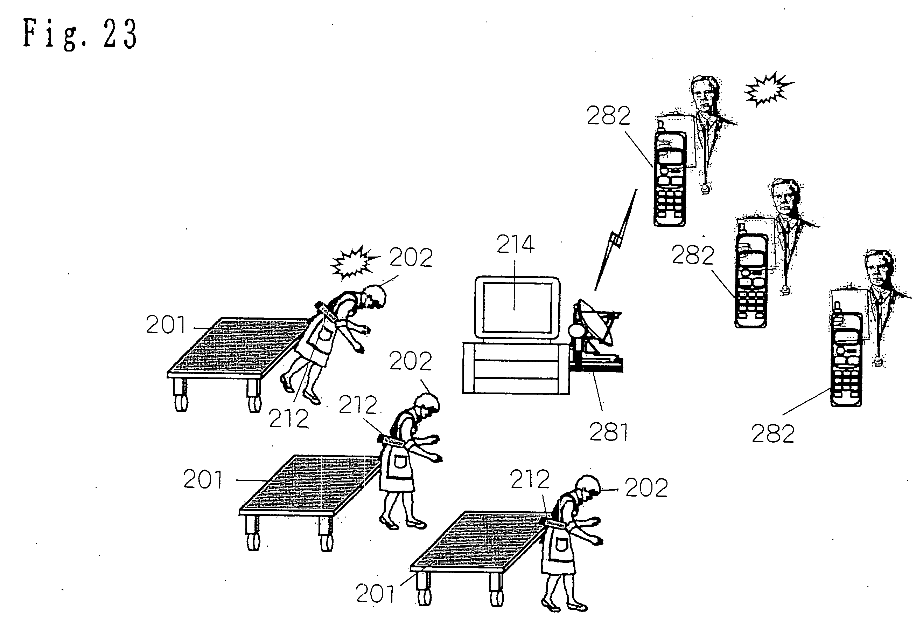

Figure 23 shows an outlined configuration of the body equilibrium state evaluation system of Embodiment 6 of the present invention.

The configuration in this Embodiment will be described below.

Figure 23 shows use in a plurality of users using cellular phones 282 as information receiving means. In this Embodiment, a plurality of cellular phones 282 are set as system information receiving means 272 shown in Figure 22. Components same as those of Embodiment 4 and 5 are given like reference symbols, and descriptions thereof are not presented.

The management terminal 214 makes a determination from information about the body equilibrium state obtained from the posture sensor 212 of each subject 202 wearing the posture sensor 212, and information sending means 81 sends the result separately to the number of the cellular phone 282 possessed by the doctor in charge or the family, which each user has previously registered.

If, for example, a unique ring tone or vibration function is used in the cellular phone 282 receiving such information, a situation of the user can be remotely known, and the advance of rehabilitation can be known, for example.

Furthermore, a program according to the above related invention is a program of making a computer perform a function of entire or partial apparatus as a body equilibrium state evaluation apparatus, which evaluates an acceleration from the start of the bed departure state until predetermined time elapses from signals obtained from acceleration detecting means and bed departure detecting means in the body equilibrium state evaluation system of the present invention, the program operating in cooperation with the computer.

Furthermore, a medium according to the related invention described above is a medium carrying a program of making a computer perform a function of entire or partial apparatus as a body equilibrium state evaluation apparatus, which evaluates an acceleration from the start of the bed departure state until predetermined time elapses from signals obtained from acceleration detecting means and bed departure detecting means in the body equilibrium state evaluation system of the present invention, wherein the medium is computer-readable, and the program read performs the functions in cooperation with the computer.

Furthermore, the "partial apparatus" of the invention refers to partial means of one apparatus or partial functions of one means.

As described above, a first related invention is a body equilibrium state evaluation method comprising a step of detecting a bed departure state in which the body of a subject departs from a bed, a step of detecting an acceleration applied to the body by acceleration detecting means attached to the body, and a step of evaluating the acceleration from the start of the bed departure state until predetermined time elapses.

A second related invention is a body equilibrium state evaluation system comprising bed departure detecting means of detecting a state in which the body of a subject departs from a bed, acceleration detecting means attached to the body and detecting an acceleration applied to the body, and a body equilibrium state evaluation/management apparatus of evaluating the acceleration from the start of the bed departure state until predetermined time elapses from signals obtained from the acceleration detecting means and the bed departure detecting means.

A third related invention is a body equilibrium state evaluation/management apparatus connected to bed departure detecting means of detecting a state in which the body of a subj ect departs from a bed, connected to acceleration detecting means attached to the body and detecting an acceleration applied to the body, and evaluating the acceleration from the start of the bed departure state until predetermined time elapses from signals obtained from the bed departure detecting means and the acceleration detecting means.

A fourth related invention is the body equilibrium state evaluation/management apparatus of the third related invention, wherein the bed departure detecting means comprises a load sensor detecting a load applied to a bed, the start of the bed departure state corresponds to a time point at which the load sensor detects a load equal to or smaller than a first predetermined value smaller than a load when the body is in an in-bed state, and the acceleration is evaluated if the load sensor detects continuously a load equal to or smaller than the first predetermined value or detects a load equal to or greater than a second predetermined value greater than a load when the body is in the in-bed state during a time interval from the start of the bed departure state until the predetermined time elapses.

A fifth related invention is the body equilibrium state evaluation/management apparatus of the third or fourth related invention, wherein the acceleration detecting means detects a first acceleration being an acceleration in a body axis direction of the body and a second acceleration being an acceleration in a direction orthogonal to the body axis direction of the body, and detects an acceleration in the gravity direction from the first acceleration and the second acceleration detected.

A sixth related invention is the body equilibrium state evaluation/management apparatus of any of the third to fifth related inventions, wherein signals obtained from the acceleration detecting means from the start of the bed departure state until the predetermined time elapses are integrated to evaluate the state of the body.

A seventh related invention is the body equilibrium state evaluation/management apparatus of the sixth related invention, wherein the integrated signals are acquired every day, and the acquired signals are evaluated with time to evaluate the state of the body.

An eighth related invention is a program of making a computer function as a body equilibrium state evaluation/management apparatus of evaluating the acceleration from the start of the bed departure state until the predetermined time elapses from signals obtained from the acceleration detecting means and the bed departure detecting means in the body evaluation/management system of the second related invention.

A ninth related invention is a medium carrying the program of the eighth related invention, the medium being capable of used with a computer.

Furthermore, one usage form of the program of the invention may be such that the program is recorded in a computer readable recording medium, and operates in cooperation with a computer.

Furthermore, one usage form of the program of the invention may be such that the program is transmitted through a transmission medium, is read by a computer, and operated in cooperation with the computer.

Furthermore, recording media include ROMs, and transmission media include transmission media such as the internet, and light/electric waves/sonic waves.

Furthermore, the computer of the invention described above may include firmware, an OS and peripheral devices, other than pure hardware such as a CPU.

Furthermore, as described above, the configuration described above may be achieved software-wise or may be achieved hardware-wise.

Furthermore, according to the invention described above, acceleration information acquired by the acceleration sensor 231 (or acceleration detecting means 12) is collected by the management terminal 214 (or calculating means 13), data within a predetermined time period is integrated, and the acceleration information is ranked, or evaluated by evaluating means 131.

Consequently, an acceleration sensor, for which downsizing, improvement in accuracy and cost-reduction have been promoted in recent years, is used, whereby the system can be more compact and inexpensive, and the sensor is attached to a trunk such as a lumbar part, whereby acquired data can be evaluated as data associated with an actual equilibrium state, thus making it possible to ensure reliability of acquired information.

Furthermore, the invention described above functions effectively in terms of general health management in that the user or doctor in charge is notified of the result of calculation.

Furthermore, according to the invention described above, in order that data produced with a motion of a subject can be acquired under certain conditions, for example, a time of getting up (or a start point and an end point of rehabilitation using parallel bars or the like) is automatically detected to collect and evaluate data.

Consequently, data collection can be achieved under certain conditions, thus making it possible to objectively evaluate post-disease physical conditions under certain conditions.

The invention described above is to propose a system of evaluating an equilibrium state of the body of a subject or a body motion of the subj ect. Furthermore, in this evaluation, for example, by detecting a time of getting up, data based on certain conditions can be automatically collected, thus making it possible to make a correct evaluation.

Consequently, a motion function state and a recovery state of physical conditions of the subject after rehabilitation, disease or the like, for which no effective methods have been available, can be conveniently and reliably managed.

Furthermore, all or part of the various kinds of functions of the posture sensor 212, the management terminal 214 and the like may be achieved by acceleration detecting means 12, calculating means 13, calculation controlling means 14, evaluating means 132 and the like included in the body motion evaluation apparatus 11 described above.

### Industrial Applicability

A body motion evaluation apparatus and a body motion evaluation system according to the present invention have an advantage that a motion function or the like of a subject can be easily and accurately evaluated compared with conventional techniques, and they are useful, for example, as a system of conveniently managing a recovery state of a motion function or the like of a subject after rehabilitation.

## Claims

1. A body motion evaluation apparatus capable of being attached to a human body, comprising:
acceleration detecting means of detecting information about an acceleration produced with a motion of a human body;
calculating means of performing predetermined calculation based on said information about the acceleration detected by said acceleration detecting means;
calculation controlling means of setting a predetermined calculation time period in said calculation by said calculating means; and
signal receiving means of receiving from outside a first behavior detection signal including information about detection of a start or end of a first predetermined behavior of said human body,
wherein said calculation controlling means determines a start point of said calculation based on said first behavior detection signal received by said signal receiving means, and sets as said calculation time period a time period from said start point until predetermined time elapses.

2. The body motion evaluation apparatus according to claim 1, comprising controlling means of controlling said acceleration detecting means, wherein said controlling means turns on the power of said acceleration detecting means at a time when said first behavior detection signal is detected by said signal receiving means, and turns off the power of said acceleration detecting means after predetermined time elapses.

3. The body motion evaluation apparatus according to claim 2, wherein said acceleration detecting means detects
(1) a first acceleration being an acceleration in a body axis direction of said human body; and
(2) at least any one of a second acceleration being an acceleration orthogonal to said body axis direction and in a front direction of said human body, and a third acceleration orthogonal to both the body axis direction of said human body and the front direction of the human body.

4. The body motion evaluation apparatus according to claim 3, wherein said acceleration detecting means detects an acceleration direction of gravity based on said first acceleration and said second acceleration detected.

5. The body motion evaluation apparatus according to claim 4, wherein said calculating means calculates the result of integration of a variation in a signal obtained by said acceleration detecting means, or a period of variation in said signal.

6. A body motion evaluation system comprising:
first behavior detecting means of detecting a start or end of said first predetermined behavior of a human body, and outputting said first behavior detection signal; and
the body motion evaluation apparatus according to claim 1 or claim 2.

7. The body motion evaluation system according to claim 6, wherein said first behavior detecting means is infrared light projecting means or electric field generating means, and
said signal receiving means is (1) infrared light receiving means or (2) electric field receiving means and signal demodulating means.

8. The body motion evaluation system according to claim 7, wherein said first behavior detecting means comprises
(1) signal sending means of sending said first behavior detection signal; and
(2) passage detecting means of detecting a passage state in which an object passes through a predetermined detection range, door opening/closing means of detecting open/close of a predetermined door, or load detecting means of detecting a load of an object at a predetermined location.

9. The body motion evaluation system according to claim 8, wherein said load detecting means is departure/seating detecting means of detecting a departure state in which an obj ect departs from a toilet seat, or detecting a seating state in which said object is seated on said toilet seat.

10. The body motion evaluation system according to claim 9, wherein said departure/seating detecting means comprises
(1) a load sensor detecting a load applied to said toilet seat;
(2) departure/seating state determining means of determining a departure state from a load detected by said load sensor.

11. The body motion evaluation system according to claim 10, wherein said departure/seating state determining means
(1) makes a determination as said departure state at a time when the load detected by said load sensor is detected as a load equal to or smaller than a first predetermined value, or
(2) makes a determination as said departure state at a time when the load detected by said load sensor is detected as a load equal to or smaller than the first predetermined value, and predetermined time elapses.

12. The body motion evaluation system according to claim 11, wherein said departure/seating state determining means determines that said object is in a seating sate at a time when it is detected that the load detected by said load sensor is equal to or greater than the first predetermined value.
